# EUROPEAN PATENT APPLICATION

(11) **EP 4 631 521 A1**
(43) Date of publication of application: **15.10.2025**
(21) Application number: 23899665.6
(22) Date of filing: 01.11.2023
(51) Int. Cl.: A61K 39/395, A61P 35/00

(54) **CANCER COMBINATION THERAPY USING CD24 ANTIBODY AND PD-1-PD-L1 PATHWAY BLOCKING ANTIBODY**

(30) Priority: 07.12.2022 CN 202211568698
(71) Applicant: ImmuneOnco Biopharmaceuticals (Shanghai) Inc., Shanghai 201203 (CN)
(72) Inventor: TIAN, Wenzhi, Shanghai 201203 (CN); LI, Song, Shanghai 201203 (CN); CHEN, Dianze, Shanghai 201203 (CN)
(74) Representative: Krauss, Jan
(86) International application number: PCT/CN2023/129029
(87) International publication number: WO 2024/120084

(57) **Abstract**

The present application relates to a composition comprising i) an antibody or an antigen-binding portion thereof that binds specifically to CD24, and ii) an antibody or an antigen-binding portion thereof that is capable of blocking PD-1-PD-L1 binding/interaction, and to the use of the composition in cancer treatment. The present application also relates to cancer combination therapies using i) an antibody or an antigen-binding portion thereof that binds specifically to CD24, and ii) an antibody or an antigen-binding portion thereof that is capable of blocking PD-1-PD-L1 binding/interaction.

## Description

This application claims priority to Chinese patent application 202211568698.8 filed on December 7, 2022.

### FIELD OF THE INVENTION

The present application relates to the combination use of an anti-CD24 antibody or an antigen-binding portion thereof, and an antibody or an antigen-binding portion thereof capable of blocking PD-1-PD-L1 binding/interaction in cancer treatment. Specifically, the present application relates to a composition that comprises i) an antibody or an antigen-binding portion thereof that binds specifically to CD24, and ii) an antibody or an antigen-binding portion thereof that is capable of blocking PD-1-PD-L1 binding/interaction, and to the use of the composition in cancer treatment and/or in the preparation of a medicament for cancer treatment.

### BACKGROUND OF THE INVENTION

Cancer cells have developed a number of mechanisms to evade host immune surveillance, including 1) to evade immune surveillance by macrophages, T lymphocytes, B lymphocytes and natural killer cells by highly expressing the membrane protein CD24, which binds to the receptors like Siglec-10 on the surface of immune cells, thereby inhibiting immune activation; 2) to evade immune surveillance by expressing high levels of the membrane proteins PD-L1 and PD-L2, which bind to the PD-1 on the surface of immune cells such as T cells, triggering apoptosis of immune cells. As can be seen, cancer cells are quite smart and can proliferate rapidly based on the evasion mechanisms they have developed.

### CD24 and Siglec-10

Sialic acid-binding immunoglobulin-like lectin (Siglec) is an immunoglobulin-like type I transmembrane protein. Among the Siglec family, Siglec-10 is an inhibitory receptor that is widely expressed on immune cells such as macrophages, B cells, NK cells and activated T cells. It has five extracellular Ig-like domains, a transmembrane region and a cytoplasmic tail. The IgV domain of Siglec-10 contains a key arginine residue that is implicated in sialic acid recognition. The Siglec-10 expression on T cells is known to interfere with T cell activation by inhibiting the formation of the T cell-major histocompatibility complex class I (MHC-I)-peptide complex and the phosphorylation of the T cell receptor-associated kinases, Lck and ZAP-70. The Siglec-10 expressed on B cells and NK cells is able to inhibit BCR-mediated and NK cell receptor-mediated signaling (Yin, et al., (2020) Front Immunol 11:1324).

CD24 is a glycosyl-phosphatidylinositol-anchored protein, found on the surfaces of developing T lymphocytes and most B lymphocytes (Yin *et al., supra*)*.* Many cancer cells, including those of ovarian cancer, breast cancer, cervical cancer, endometrial cancer, colon cancer, non-small cell lung cancer, small cell lung cancer, head and neck tumor, uroepithelial cancer, acute lymphocytic leukemia (ALL), acute myeloid leukemia (AML), multiple myeloma, Hodgkin's lymphoma (HL), non-Hodgkin's lymphoma (NHL), cholangiocarcinomas, liver cancer, bladder cancer, pancreatic cancer, gastric cancer and glioblastoma, also highly express CD24 (Barkal et al., (2019) Nature 572(7769): 392-396; Liu et al., (2013) Oncol Lett 6(1): 96-100; Panagiotou E et al., (2022) J Pers Med. 12(8):1235). The CD24 on cancer cells interacts with the Siglec-10 on immune cells, to generate "don't eat me" signals that are used for immune evasion and protect tumor cells from immune attacks, including to inhibit phagocytosis by macrophages.

Studies have shown a correlation between CD24 expression and bladder tumor recurrence (Liu *et al., supra*)*.* In patients of ovarian cancer, CD24 expression is an independent predictor of overall survival, and correlates with tumor stage, peritoneal and lymph node metastasis; CD24-positive cells have enhanced proliferation, a highly invasive phenotype, and are associated with cisplatin resistance in ovarian cancer cells (Nakamura et al., (2017) Oncol Rep. 37(6):3189-3200). A monoclonal anti-CD24 antibody was found to reduce lung metastases and prolong overall survival in mouse models of bladder cancer and triple-negative breast cancer. The literatures further showed that, to block the CD24-Siglec 10 interaction by antibodies, can induce macrophage-mediated tumor growth reduction and prolong survival of tumor-bearing mice (Barkal, *et al., supra;* Chan et al., (2019) Mol Cancer Ther 18(1): 147-161; Overdevest et al., (2011) Cancer Res 71(11): 3802-11). In addition, in various preclinical animal experiments, CD24 inhibitors have shown inhibitory effects on a wide range of tumor models: ovarian cancer, breast cancer, non-small cell lung cancer, small cell lung cancer, head and neck tumor, uroepithelial cancer, non-Hodgkin's lymphoma (NHL), liver cancer, bladder cancer, pancreatic cancer, gastric cancer, and colorectal cancer (Panagiotou E et al., (2022) J Pers Med. 12(8):1235).

### PD-L1 and PD-1

PD-1 is a checkpoint molecule that down-regulates the immune responses and is expressed predominantly in activated T cells and B cells. PD-L1 and PD-L2 are the two ligands of PD-1, of which PD-L1 is expressed on antigen-presenting cells, T cells, B cells, monocytes and epithelial cells, among others (Keir ME et al., (2008) Annu Rev Immunol. 26: 677-704; Chen J et al., (2016) Ann Oncol. 27(3): 409-416). The engagement of PD-L1 or PD-L2 with PD-1 transmits inhibitory signals that impede the activation of T cells and B cells, so as to achieve the homeostasis of the immune system, avoiding unintentional injury to normal cells.

However, the PD-1 signaling may be exploited by tumor cells, to help them evade immune surveillance. Studies have shown that a variety of tumor cells constitutively express PD-L1 and/or PD-L2, and that the tumor microenvironment (TME) induces immune cells such as infiltrating T cells to highly express PD-1 molecules. In such circumstances, the PD-1 signaling in the tumor microenvironment is continuously activated, which inhibits the killing of tumor cells by T cells.

Studies have suggested that blockade of PD-1 signaling may inhibit the growth of a variety of solid and hematologic tumors. The PD-1 or PD-L1 targeted indications that have been approved or are in clinical trials include melanoma, non-small cell lung cancer, renal cell carcinoma, Hodgkin's lymphoma, bladder cancer, head and neck cancer, neuroendocrine tumor, and solid tumors with high microsatellite instability and defective mismatch repair, as well as mantle cell lymphomas, diffuse large B-cell lymphomas and follicular lymphomas (Akinleye A, Rasool Z. (2019) J Hematol Oncol. 12(1): 92; Chong Sun et al., (2018) Immunity 48(3): 434-452). The anti-PD-1 antibodies currently on market include tislelizumab, nivolumab (Opdivo), or pembrolizumab (Keytruda), and the anti-PD-L1 antibodies that have been approved include atezolizumab, durvalumab, and avelumab.

### Combination therapies

Therapies targeting single molecules, such as PD-1 or PD-L1, have shown efficacy in a wide range of cancers, but a substantial portion of patients are not responsive to them. For example, some review papers mentioned that PD-1 or PD-L1 blocking antibodies have limited efficacy in ovarian cancer and pancreatic cancer (Panagiotou E *et al.,* (2022) *supra*)*,* whereas CD24 is highly expressed on ovarian cancer and pancreatic cancer (Barkal et al., (2019) Nature 572(7769): 392-396; Liu et al., (2013) Oncol Lett 6(1): 96-100).

Combined drug uses have become a new therapeutic strategy where cancer cells might be more efficiently killed to suppress cancer growth when more than one signaling pathway is targeted. However, not all drug combinations result in better therapeutic outcomes, and not all drugs can be combined in use. For example, in a pooled analysis of 14 phase I-III studies, 93% incidence of adverse reactions was found in patients receiving a dual therapy with anti-PD-1 antibodies and anti-CTLA4 antibodies, while 64% of patients receiving anti-CTLA-4 antibodies alone at multiple doses experienced immune-related adverse reactions (Wolchok, J. D. et al., (2013) N. Engl. J. Med. 369:122-33). As another example, the combination use of panobinostat and carfilzomib caused treatment-related heart failure (2%) and a 2% increase in treatment-related mortality in patients with relapsed/refractory multiple myeloma (Berdeja JG et al., (2015) Haematologica 100(5): 670-676).

In addition, till now, the interaction between and the role of the two immune checkpoint pathways, CD24/Siglec-10 and PD-1/PD-L1, remain to be elucidated, and more data is needed to support the presence of any anti-cancer effect of the combination use of agents antagonizing the two axes, CD24/Siglec-10 and PD-1/PD-L1 (Panagiotou *E et al.,* (2022) *supra*)*.* Interestingly, in non-small cell lung cancer with relatively low PD-L1 expression, CD24 positivity seemed to be negatively correlated with the progression-free survival of patients who had been treated with immune checkpoint inhibitors (Ozawa Y, et al., (2021) Cancer Sci. 112(1): 72-80).

Citation or identification of any document in this application is not an admission that such document is available as prior art to the present invention.

### SUMMARY OF THE INVENTION

The inventors of the present application, surprisingly found that, targeting the CD24 pathway and the PD-1-PD-L1 pathway simultaneously, e.g., using an antibody specifically binding CD24 in combination with an antagonistic anti-PD-1 or anti-PD-L1 antibody, may suppress tumor or cancer growth in a synergistic manner.

In a first aspect, the present application provides a method of treating cancer, comprising administering to a subject i) an antibody or an antigen-binding portion thereof that binds specifically to CD24, and ii) an antibody or an antigen-binding portion thereof that is capable of blocking PD-1-PD-L1 binding or PD-1-PD-L1 interaction.

The antibody or antigen-binding portion thereof that binds specifically to CD24 may specifically bind to CD24 (e.g., human CD24) and may preferably block CD24-Siglec binding or CD24-Siglec interaction. In some embodiments, the antibody or antigen-binding portion thereof that binds specifically to CD24 may comprise an FcR-binding heavy chain constant region, to trigger an antibody-dependent cell-mediated cytotoxic effect (ADCC) against CD24⁺ tumor cells.

In some embodiments, the antibody or antigen-binding portion thereof that binds specifically to CD24 may comprise a heavy chain variable region and a light chain variable region, wherein the heavy chain variable region may comprise a heavy chain variable region CDR1 (HV-CDR1), a HV-CDR2, and a HV-CDR3, and the light chain variable region may comprise a light chain variable region CDR1 (LV-CDR1), a LV-CDR2, and a LV-CDR3, wherein the HV-CDR1, the HV-CDR2, and the HV-CDR3 may comprise amino acid sequences having at least 85%, 86%, 87%, 88%, 89%, 90%, 91%, 92%, 93%, 94%, 95%, 96%, 97%, 98%, 99% or 100% sequence identity to GYSITSGYS (SEQ ID NO: 1), IHYSGST (SEQ ID NO: 2) and ARGADYALDY (SEQ ID NO: 3), respectively, and the LV-CDR1, the LV-CDR2, and the LV-CDR3 may comprise amino acid sequences having at least 85%, 86%, 87%, 88%, 89%, 90%, 91%, 92%, 93%, 94%, 95%, 96%, 97%, 98%, 99% or 100% sequence identity to QSLLYSSNQKNY (SEQ ID NO: 4), WAS, and QQNFIYPLT (SEQ ID NO: 5), respectively. In certain embodiments, the heavy chain variable region and the light chain variable region may comprise amino acid sequences having at least 85%, 86%, 87%, 88%, 89%, 90%, 91%, 92%, 93%, 94%, 95%, 96%, 97%, 98%, 99% or 100% sequence identity to 1) SEQ ID NOs: 6 and 7, respectively; 2) SEQ ID NOs: 6 and 10, respectively; or 3) SEQ ID NOs: 11 and 12, respectively. In one embodiment, the heavy chain variable region and the light chain variable region may comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 6 and 7, respectively. The antibody or antigen-binding portion thereof that binds specifically to CD24 may further comprise a heavy chain constant region and/or a light chain constant region. The heavy chain constant region may comprise FcR binding ability, to trigger e.g., ADCC. In one embodiment, the heavy chain constant region may be, e.g., a human IgG1 heavy chain constant region comprising e.g., the amino acid sequence of SEQ ID NO: 8. The light chain constant region may be, for example, a human κ light chain constant region, comprising e.g., the amino acid sequence of SEQ ID NO: 9.

The antibody or antigen-binding portion thereof capable of blocking PD-1-PD-L1 binding or PD-1-PD-L1 interaction may be an antagonistic anti-PD-1 antibody or an antigen-binding portion thereof capable of binding specifically to PD-1, or an antagonistic anti-PD-L1 antibody or an antigen-binding portion thereof capable of binding specifically to PD-L1.

The antagonistic anti-PD-1 antibody or antigen-binding portion thereof may bind specifically to PD-1 (e.g., human PD-1) and preferably block PD-1-PD-L1 binding or interaction, or alternatively PD-1-PD-L1 and PD-1-PD-L2 bindings or interactions. In certain embodiments, the antagonistic anti-PD-1 antibody or antigen-binding portion thereof can be tislelizumab, nivolumab (e.g., Opdivo^{®}), pembrolizumab (e.g., Keytruda^{®}), or an antigen-binding portion thereof.

The antagonistic anti-PD-L1 antibody or antigen-binding portion thereof may specifically bind to PD-L1 (e.g., human PD-L1) and preferably block PD-1-PD-L1 binding or PD-1-PD-L1 interaction. In certain embodiments, the antagonistic anti-PD-L1 antibody or antigen-binding portion thereof may comprise a heavy chain constant region capable of binding FcR, to, e.g., trigger ADCC against PD-L1⁺ tumor cells. The antagonistic anti-PD-L1 antibody or antigen-binding portion thereof may be atezolizumab (e.g., Tecentriq^{®}), durvalumab, avelumab, IMM2515H, or an antigen-binding portion thereof.

IMM2515H may comprise a heavy chain variable region and a light chain variable region, wherein the heavy chain variable region may comprise a heavy chain variable region CDR1 (HV-CDR1), a HV-CDR2, and a HV-CDR3, and the light chain variable region may comprise a light chain variable region CDR1 (LV-CDR1), a LV-CDR2, and a LV-CDR3, wherein the HV-CDR1, the HV-CDR2, and the HV-CDR3 may comprise amino acid sequences having at least 85%, 86%, 87%, 88%, 89%, 90%, 91%, 92%, 93%, 94%, 95%, 96%, 97%, 98%, 99% or 100% sequence identity to GYTFTSNW (SEQ ID NO: 13), IHPNSGSS (SEQ ID NO: 14), and ARSYYGSSPYYFDY (SEQ ID NO: 15), respectively, and the LV-CDR1, the LV-CDR2, and the LV-CDR3 may comprise amino acid sequences having at least 85%, 86%, 87%, 88%, 89%, 90%, 91%, 92%, 93%, 94%, 95%, 96%, 97%, 98%, 99% or 100% sequence identity to QDIINY (SEQ ID NO: 16), YTS and QQGDTLPWT (SEQ ID NO: 17), respectively. In certain embodiments, the heavy chain variable region and the light chain variable region may comprise amino acid sequences having at least 85%, 86%, 87%, 88%, 89%, 90%, 91%, 92%, 93%, 94%, 95%, 96%, 97%, 98%, 99% or 100% sequence identity to SEQ ID NOs: 18 and 19, respectively. IMM2515H may further comprise a heavy chain constant region and/or a light chain constant region. The heavy chain constant region may comprise FcR binding ability, to trigger e.g., ADCC. In one embodiment, the heavy chain constant region may be, e.g., a human IgG1 heavy chain constant region comprising e.g., the amino acid sequence of SEQ ID NO: 8. The light chain constant region may be, for example, a human κ light chain constant region, comprising e.g., the amino acid sequence of SEQ ID NO: 9.

The method may comprise simultaneously administering i) an antibody or an antigen-binding portion thereof that binds specifically to CD24, and ii) an antibody or an antigen-binding portion thereof that is capable of blocking PD-1-PD-L1 binding or PD-1-PD-L1 interaction, or sequentially administering i) an antibody or an antigen-binding portion thereof that binds specifically to CD24, and ii) an antibody or an antigen-binding portion thereof that is capable of blocking PD-1-PD-L1 binding or PD-1-PD-L1 interaction.

In some embodiments, the cancer may be a solid cancer or a hematologic cancer, including, but not limited to, gastric cancer, liver cancer, colon cancer, non-small cell lung cancer, small cell lung cancer, ovarian cancer, pancreatic cancer, breast cancer, cervical cancer, endometrial cancer, colon cancer, head and neck tumor, uroepithelial carcinoma, cholangiocarcinoma, bladder cancer, glioblastoma, acute lymphocytic leukemia (ALL), acute myeloid leukemia (AML), multiple myeloma, Hodgkin's lymphoma (HL), and non-Hodgkin's lymphoma (NHL). In some embodiments, the cancer may be a CD24-expressing cancer. In some embodiments, the cancer may be a cancer that does not express CD24. In some embodiments, the cancer may be a cancer that expresses PD-L1 and/or PD-L2. In some embodiments, the cancer may be a cancer that does not express PD-L1 or PD-L2. In some embodiments, the cancer may be a cancer that highly expresses PD-L1. In some embodiments, the cancer may be a cancer with low PD-L1 expression. In some embodiments, the cancer may be colon cancer.

The present application also relates to the use of i) an antibody or an antigen-binding portion thereof that bind specifically to CD24, and ii) an antibody or an antigen-binding portion thereof capable of blocking PD-1-PD-L1 binding or PD-1-PD-L1 interaction in cancer treatment, and in the preparation of a medicament for treating cancer.

In a second aspect, the present application provides a composition, such as a pharmaceutical composition, that comprises i) an antibody or an antigen-binding portion thereof that binds specifically to CD24, and ii) an antibody or an antigen-binding portion thereof that is able to block PD-1-PD-L1 binding or PD-1-PD-L1 interaction.

The antibody or antigen-binding portion thereof that binds specifically to CD24, and the antibody or antigen-binding portion thereof that is capable of blocking PD-1-PD-L1 binding or PD-1-PD-L1 interaction are as defined above.

In one embodiment, a pharmaceutical composition is provided that comprises a therapeutically effective amount of i) an antibody or an antigen-binding portion thereof that binds specifically to CD24, and ii) an antibody or an antigen-binding portion thereof that is capable of blocking PD-1-PD-L1 binding or PD-1-PD-L1 interaction. The pharmaceutical composition may further comprise a pharmaceutically acceptable carrier.

In a third aspect, the present application provides a method of treating cancer in a subject in need thereof, comprising administering to the subject i) an antibody or an antigen-binding portion thereof that binds specifically to CD24, and ii) an antibody or an antigen-binding portion thereof capable of blocking PD-1-PD-L1 binding or PD-1-PD-L1 interaction. In some embodiments, the method comprises administering to the subject a composition of the present application.

The cancer may be a solid cancer or a hematologic cancer, including, but not limited to, gastric cancer, liver cancer, colon cancer, non-small cell lung cancer, small cell lung cancer, ovarian cancer, pancreatic cancer, breast cancer, cervical cancer, endometrial cancer, head and neck tumor, uroepithelial carcinoma, cholangiocarcinoma, bladder cancer, glioblastoma, acute lymphocytic leukemia (ALL), acute myeloid leukemia (AML), multiple myeloma, Hodgkin's lymphoma (HL), and non-Hodgkin's lymphoma (NHL). In some embodiments, the cancer may be a cancer that expresses CD24, PD-L1 and/or PD-L2, or a cancer that does not express CD24, PD-L1 or PD-L2. In some embodiments, the cancer may be a cancer that highly expresses PD-L1. In some embodiments, the cancer may be a cancer with low PD-L1 expression. In some embodiments, the cancer may be colon cancer.

The present application also relates to the use of the composition of the present application in cancer treatment, and in the preparation of a medicament for treating cancer.

Other features and advantages of the instant disclosure will be apparent from the following detailed description and examples, which should not be construed as limiting. All references, Genbank entries, patents and published patent applications cited throughout this application are incorporated herein by reference.

### BRIEF DESCRIPTION OF THE DRAWINGS

The following detailed description, given by way of example, but not intended to limit the current application solely to the specific embodiments as described, may best be understood with reference to the accompanying drawings.
FIG. 1 shows the average tumor size changes in each group of mice treated by IMM47 and tislelizumab.
FIG. 2 shows the tumor size changes of individual mice from each group treated by IMM47 and tislelizumab.
FIG. 3 shows the average tumor size changes in each group of mice treated by IMM47 in combination with Opdivo^{®} or Keytruda^{®}.
FIG. 4 shows the tumor size changes of individual mice from each group treated by IMM47 in combination with Opdivo^{®} or Keytruda^{®}.

### DETAILED DESCRIPTION OF THE INVENTION

Before disclosing and describing particular embodiments of the present application, it should be understood that the present invention is not limited to the particular methods and materials disclosed herein, and that these may vary to some extent. It should be understood that the terminologies used herein are for the purpose of describing particular embodiments only and is not limiting, as the scope of this application will be limited only by the appended claims and their equivalents.

The term "antibody" as used herein includes, for example, whole antibodies of IgG, IgA, IgD, IgE and IgM, and any antigen-binding fragment (or antigen-binding portion) or single chains thereof. A whole antibody is a glycoprotein comprising at least two heavy chains and two light chains, the heavy chain and the light chain being linked by disulfide bond(s). Each heavy chain contains a heavy chain variable region (V_{H}) and a heavy chain constant region. The heavy chain constant region contains three domains, C_{H1}, C_{H2}, and C_{H3}. Each light chain contains a light chain variable region (V_{L}) and a light chain constant region. The light chain constant region contains one domain, C_{L}. The V_{H} and V_{L} regions can be further subdivided into regions of hypervariability, termed complementarity determining regions, interspersed with regions that are more conserved, termed framework regions (FR). Each V_{H} or V_{L} is composed of three CDRs and four FRs, arranged from amino-terminus to carboxyterminus in the following order: FR1, CDR1, FR2, CDR2, FR3, CDR3, FR4. The variable regions of the heavy and light chains contain a binding domain that interacts with an antigen. The constant regions of an antibody may mediate the binding of the immunoglobulin to host tissues or factors, including various cells of the immune system (e.g., effector cells) and the first component (C1q) of the classical complement system.

The term "antigen-binding portion" of an antibody (or simply "antibody portion") refers to one or more fragments of an antibody that retain the ability to specifically bind to an antigen. It has been shown that the antigen-binding function of an antibody can be performed by fragments of a full-length antibody. Examples of the binding fragments encompassed within the term "antigen-binding portion" of an antibody include (i) a Fab fragment, a monovalent fragment consisting of the V_{L}, V_{H}, C_{L} and C_{H1} domains; (ii) a F(ab')₂ fragment, a bivalent fragment which comprises two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment, which consists of a V_{H} domain (Ward et al., (1989) Nature 341: 544-546); and (vi) an isolated complementarity determining region (CDR). Furthermore, although the two domains of the Fv fragment, V_{L} and V_{H}, are coded by separate genes, they can be joined, using recombinant methods, by a synthetic linker, wherein the synthetic linker enables them to be made as a single protein chain in which the V_{L} and V_{H} regions pair to form a monovalent molecule (known as single chain Fv (scFv)). Such single chain antibodies are also intended to be encompassed within the term "antigen-binding portion" of an antibody. These antibody fragments are obtained using conventional techniques known to those with skill in the art, and the fragments are screened for utility in the same manner as are intact antibodies.

The heavy chain variable region CDRs and the light chain variable region CDRs of the antibody or antigen-binding portion thereof of the disclosure have been defined by the IMGT numbering system. As is well known in the art, the heavy chain variable region and light chain variable region CDRs can be determined by e.g., Chothia, Kabat, AbM or Contact numbering system/method.

The term "mouse" heavy/light chain variable region used herein refers to a variable region whose framework (FR) and CDR regions are all derived from mouse germline immunoglobulin sequences, while a "humanized" heavy/light chain variable region refers to a variable region that is derived from a non-human species but has been modified to increase similarity to antibody variants produced naturally in humans.

The term "an antibody or an antigen-binding portion thereof capable of blocking PD-1-PD-L1 binding/interaction" refers to any antibody or the antigen-binding portion thereof capable of blocking PD-1-PD-L1 binding or PD-1-PD-L1 interaction, to thereby block or inhibit the PD-1 signaling pathway, including, but not limited to, an anti-PD-1 antibody or an antigen-binding portion thereof that binds specifically to PD-1 and blocks PD-1-PD-L1 interaction, an anti-PD-L1 antibody or an antigen-binding portion thereof that binds specifically to PD-L1 and blocks PD-1-PD-L1 interaction, an anti-PD-L1 antibody or an antigen-binding portion thereof that binds specifically to PD-L1 and achieves PD-1-PD-L1 blocking by inducing ADCC or the like to kill PD-L1 cells.

The term "antagonistic" or "blocking" antibody or antigen-binding portion thereof refers to an antibody or an antigen-binding portion thereof that specifically binds a specified antigen and blocks this antigen-mediated signaling pathway(s). For example, an antagonistic anti-PD-1 antibody or an antigen-binding portion thereof, refers to an antibody or an antigen-binding portion thereof that specifically binds PD-1 and blocks the PD-1 signaling pathway(s) induced by the binding of e.g., PD-L1, or PD-L1 plus PD-L2 to PD-1; an antagonistic anti-PD-L1 antibody or an antigen-binding portion thereof, refers to an antibody or an antigen-binding portion thereof that specifically binds PD-L1 and blocks the PD-1 signaling pathway(s) induced by PD-1-PD-L1 interaction, including an anti-PD-L1 antibody or an antigen-binding portion thereof that binds specifically to PD-L1 and blocks PD-1-PD-L1 interaction, an anti-PD-L1 antibody or an antigen-binding portion thereof that binds specifically to PD-L1 and achieves PD-1-PD-L1 blocking by inducing ADCC or the like to kill PD-L1 cells.

The "high expression" or "low expression" of PD-L1 as mentioned in the present application may be determined according to the criteria known in the art, or the criteria mentioned in Ozawa Y, et al. (2021) Cancer Sci. 112(1):72-80. The cells used in the Examples to construct the mouse tumor models were with "high expression" of PD-L1 relative to common PD-L1-expressing tumor cells.

The term "antibody-dependent cytotoxicity", "antibody-dependent cell-mediated cytotoxicity" or "ADCC" refers to cell-mediated immune defense in which the effector cells of the immune system actively lyse target cells such as cancer cells whose membrane surface antigens have been bound by antibodies such as anti-CD24 antibodies.

The term "sequence identity" as used herein refers to the percentage of nucleotides/amino acids in a sequence that are identical to the nucleotides/amino acid residues in a reference sequence after sequence alignment, and if necessary, spaces are introduced in the sequence alignment to achieve the maximum percentage of sequence identity between the two sequences. Those skilled in the art can perform pairwise sequence alignment or multiple sequence alignment to determine the percentage of sequence identity between two or more nucleic acid or amino acid sequences by a variety of methods, such as using computer software, such as ClustalOmega, T-coffee, Kalign, MAFFT, and etc.

The term "subject" includes any human or non-human animal. The term "non-human animal" includes all vertebrates, e.g., mammals and non-mammals, such as non-human primates, sheep, dogs, cats, cows, horses, chickens, amphibians, and reptiles, although mammals are preferred, such as non-human primates, sheep, dogs, cats, cows and horses.

The phrase "pharmaceutically acceptable" is used herein to refer to those compounds, materials, compositions, and/or dosage forms which are, within the scope of sound medical judgment, suitable for use in contact with human and animal tissues without excessive toxicity, irritation, allergic reactions, and/or other problems or complications, and meet reasonable benefit/risk ratios.

As used herein, the term "effective amount" means, for example, an amount of a medicament or pharmaceutical agent, such as an antibody or an antigen-binding portion thereof or a composition of the present application, that is being sought by an investigator or a clinician to elicit a biological or medical response in a tissue, system, animal, or human. In addition, the term "therapeutically effective amount" means any amount that, as compared to a counterpart subject who did not receive such amount, results in improved treatment, cure, prevention, reduction of a disease, condition or side effects, or a decrease in the rate of advancement of a disease or condition. An effective amount may be administered in one or more administrations, applications, or dosages, and is not intended to be limited to a particular formulation or route of administration. For example, a "therapeutically effective amount" of a composition or an antibody combination use of the present application preferably results in a decrease in severity of disease symptoms, an increase in frequency and duration of disease symptom-free periods, or a prevention of impairment or disability due to the disease affliction. For example, for the treatment of tumor-bearing subjects, a "therapeutically effective amount" inhibits tumor growth by at least about 40%, more preferably by at least about 60%, more preferably by at least about 80%, more preferably by at least about 99% relative to untreated subjects.

As used herein, the term "treatment" includes any effect, e.g., lessening, reducing, modulating, ameliorating or eliminating, that results in the improvement of the condition, disease, disorder, and the like, or amelioration of a symptom thereof.

As used herein, the term "pharmaceutical composition" refers to a combination of an active agent and a carrier (inert or active) that makes the composition particularly suitable for diagnostic or therapeutic use *in vivo* or *in vitro.*

### Combination therapy

The present application provides a combination therapy for cancer, comprising administering to a subject i) an antibody or an antigen-binding portion thereof that binds specifically to CD24, and ii) an antibody or an antigen-binding portion thereof that is capable of blocking PD-1-PD-L1 binding or interaction.

The antibody or antigen-binding portion thereof that specifically binds to CD24, in one embodiment, comprises a heavy chain variable region CDR1 (HV-CDR1), a HV-CDR2, a HV-CDR3, a light chain variable region CDR1 (LV-CDR1), a LV-CDR2, and a LV-CDR3 that comprise the amino acid sequences GYSITSGYS (SEQ ID NO: 1), IHYSGST (SEQ ID NO: 2), ARGADYALDY (SEQ ID NO: 3), QSLLYSSNQKNY (SEQ ID NO: 4), WAS, and QQNFIYPLT (SEQ ID NO: 5), respectively. It may comprise an FcR-binding heavy chain constant region, to trigger antibody-dependent cell-mediated cytotoxicity (ADCC) against CD24⁺ tumor cells. Exemplary antibodies, including IMM47, IMM47C and IMM47H, were described in Chinese patent application CN202111195246.5 and have shown their binding ability to CD24⁺ cells, ability to trigger ADCC against CD24⁺ cells, and possession of potent *in vivo* anti-tumor activity.

The antibody or antigen-binding portion thereof that is capable of blocking PD-1-PD-L1 binding or interaction refers to any antibody or the antigen-binding portion thereof that can block or inhibit the PD-1 signaling pathway, including, but not limited to, an anti-PD-1 antibody or an antigen-binding portion thereof that binds specifically to PD-1 and blocks PD-1-PD-L1 interaction, an anti-PD-L1 antibody or an antigen-binding portion thereof that binds specifically to PD-L1 and blocks PD-1-PD-L1 interaction, an anti-PD-L1 antibody that specifically binds to PD-L1 and achieves PD-1-PD-L1 blocking by inducing ADCC or the like to kill PD-L1 cells.

It is well known in the antibody field that the CDR regions are critical to an antibody's antigen binding ability, while the amino acids in the framework regions of the variable region may be modified or changed to a certain extent without altering the antibody's antigen binding capability and binding specificity.

The combination therapy of the present application may be used to treat cancer, including but not limited to colon cancer. In some embodiments, the combination therapy of the present application showed synergistic effect, with significantly better tumor killing capability compared to CD24 targeting alone, and PD-1/PD-L1 targeting alone. Moreover, the dose of each antibody may be reduced to some extent as compared to the single targeted therapies, and the combination use still show better therapeutic effects than single-targeted therapies.

The combination therapy of the present application may be applied to animals, preferably mammals (e.g., domesticated animals, cats, dogs, mice, rats), and more preferably humans. Any method of administration may be used to deliver both antibodies of the present application to a subject in need thereof. In some embodiments, the combined use of the two antibodies in this application are administered parenterally, via e.g., intraperitoneal injection, intravenous injection and the like.

One or more other agents or treatments such as other chemotherapeutic agents or other anti-cancer agents, immune enhancers, immunosuppressants, anti-tumor vaccines, and/or cytokine therapies (e.g., IL2 and GM-CSF), may optionally be used with the combination therapy of the present application. Other agents may be combined with the two antibodies of the present application in a single dosage form, or these therapeutic agents may be used as separate dosage forms.

The combination therapy of the present application can be administered concurrently as a single composition in a pharmaceutically acceptable carrier, or concurrently as separate compositions with each agent in a pharmaceutically acceptable carrier. If more than one dose of the combination therapy is administered sequentially, the order of the sequential administration can be reversed or kept in the same order at each time point of administration. The sequential administrations can be combined with concurrent administrations, or any combination thereof.

### Composition and Uses

The present application provides a pharmaceutically acceptable composition that comprises a therapeutically effective amount of an antibody or an antigen-binding portion thereof that binds specifically to CD24, formulated together with one or more pharmaceutically acceptable carriers, a therapeutically effective amount of an antibody or an antigen-binding portion thereof that is capable of blocking PD-1-PD-L1 binding or interaction, formulated together with one or more pharmaceutically acceptable carriers, and optionally, one or more other therapeutic agents as desired.

The pharmaceutical composition may comprise any number of carriers, including surfactants, thickening or emulsifying agents, solid binders, dispersion or suspension aids, solubilizers, colorants, flavoring agents, coatings, disintegrating agents, lubricants, sweeteners, preservatives, isotonic agents, and the combinations thereof.

Preferably, the pharmaceutical composition is suitable for intravenous, intramuscular, subcutaneous, parenteral, spinal or epidermal administration (e.g., by injection or infusion). Depending on the route of administration, the active ingredient can be coated in a material to protect it from the action of acids and other natural conditions that may inactivate it. The term "parenteral administration" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion. Alternatively, an composition of the disclosure can be administered via a non-parenteral route, such as a topical, epidermal or mucosal route of administration, e.g., intranasally, orally, vaginally, rectally, sublingually or topically.

The pharmaceutical compositions can be in the form of sterile aqueous solutions or dispersions. They can also be formulated in a microemulsion, liposome, or other ordered structure suitable to high drug concentration.

The dosing regimen for the pharmaceutical composition of the present application will vary depending on known factors, such as the pharmacodynamic characteristics of each particular agent, and the mode and route of administration thereof, the recipient's species, age, gender, health status, medical condition, and body weight; the nature and extent of the symptoms; the kind of concurrent treatment; the frequency of treatment; the route of administration, the patient's renal and hepatic function, and the desired effect. The amount of active ingredient which can be combined with a carrier material to produce a single dosage form will vary depending upon the subject being treated and the particular mode of administration and will generally be that amount of the composition which produces a therapeutic effect. Generally, out of one hundred percent, this amount will range from about 0.01% to about 99% of active ingredient in combination with a pharmaceutically acceptable carrier.

Dosage regimens may be adjusted to provide the optimum desired response (e.g., a therapeutic response). For example, a single bolus can be administered, several divided doses can be administered over time or the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active ingredient calculated to produce the desired therapeutic effect in association with the required pharmaceutical carrier. Alternatively, the antibodies can be administered as a sustained release formulation, in which case less frequent administration is required. The controlled release formulation includes implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. In certain embodiments, the composition of the disclosure can be formulated to ensure proper distribution *in vivo.* For example, to ensure that the composition of the disclosure cross the blood-brain barrier, it can be formulated in liposomes.

The present application also relates to a gene therapy *in vivo* wherein the nucleic acid molecule(s) encoding the two antibodies or antigen-binding portions thereof is/are introduced directly into a subject. For example, the nucleic acid sequence(s) encoding the antibodies or antigen-binding portions thereof of the disclosure is/are introduced into target cells via local injection of nucleic acid construct(s) with or without an appropriate delivery vector, such as an adeno-associated virus vector. Alternative viral vectors that can be selected include, but are not limited to, retroviruses, adenovirus, herpes simplex vims and papilloma virus vectors. The *in vivo* physical transfer of the virus vector(s) may be achieved by local injection of the desired nucleic acid construct(s) or other appropriate delivery vector(s) containing the desired nucleic acid sequence(s), liposome-mediated transfer, direct injection (naked DNA(s)), or microparticle bombardment (gene-gun).

The composition of the present application may be used to treat cancer, including, but not limited to, colon cancer. In some embodiments, the administration of the composition of the present application show a synergistic effect, with significantly better tumor killing capability compared to CD24 targeting alone, and PD-1/PD-L1 targeting alone.

The application will be further described with reference to the following nonlimiting Examples.

### Examples

IMMA47 in the present application is an IgG antibody against CD24 and contains two heavy chains and two light chains, wherein each heavy chain contains a mouse heavy chain variable region (SEQ ID NO: 6) and a human IgG1 constant region (SEQ ID NO: 8), and each light chain contains a humanized light chain variable region (SEQ ID NO: 7) and a human κ constant region (SEQ ID NO: 9).

IMM47, and two other anti-CD24 antibodies IMM47C and IMM47H with the same heavy and light chain variable region CDRs, have shown their CD24⁺ cell binding ability, their ability to trigger ADCC against CD24⁺ cells, and possession of potent *in vivo* anti-tumor activity. In particular, IMM47C contains a mouse heavy chain variable region (SEQ ID NO: 6), a human IgG1 constant region (SEQ ID NO: 8), a mouse light chain variable region (SEQ ID NO: 10), and a human κ constant region (SEQ ID NO: 9); IMM47H contains a humanized heavy chain variable region (SEQ ID NO: 11), a human IgG1 constant region ( SEQ ID NO: 8), a humanized light chain variable region (SEQ ID NO: 12) and a human κ constant region (SEQ ID NO: 9). The details can be found in Chinese patent application CN202111195246.5.

IMM2515H in this application is an IgG antibody against PD-L1, comprising two heavy chains and two light chains, wherein each heavy chain comprises a heavy chain variable region (SEQ ID NO: 18) and a human IgG1 constant region (SEQ ID NO: 8), and each light chain comprises a light chain variable region (SEQ ID NO: 19) and a human κ constant region (SEQ ID NO: 9).

### Example 1. In vivo anti-tumor efficacy of IMM47 in combination with tislelizumab

The human CD24 gene (Uniprot ID # P25063) was cloned into a neomycin resistant expression vector pMac-G418 using restriction enzyme cleavage sites HindIII and NotI, to obtain an hCD24 expression plasmid. In addition, the human PD-L1 gene (Uniprot ID # Q9NZQ7) was cloned into a puromycin-resistant expression vector pMac-Pur using restriction enzyme cleavage sites Hind III and NotI, to obtain an hPD-L1 expression plasmid.

Using the agent PEI, MC38 cells were transiently transfected with the hCD24 expression plasmid above, screened with neomycin selection pressure for polyclones stably expressing human CD24, and subcloned in 96-well plates using the limited dilution method to select stable monoclonal cell strains with high CD24 expression. Using the same protocol as above, the selected MC38 cells stably and highly expressing human CD24 were transfected with the hPD-L1 expression plasmid above, and screened using puromycin for the monoclonal cell strains capable of stably and highly expressing both human CD24 and human PD-L1.

The MC38-hCD24/hPD-L1 cells prepared as above were cultured in high-sugar DMEM medium containing 10% inactivated fetal bovine serum in an incubator at 37°C under 5% CO₂, and the cells were separated into flasks and passaged every 2 to 3 days when the confluency reached 80%.

The MC38-hCD24/hPD-L1 cells were collected at the log phase and resuspended in PBS for cell counting, and the cell density was adjusted to 3.0×10⁷/mL. The cell suspensions were inoculated subcutaneously into the right flanks of 6-8-week-old C57BL/6-hPD-1 mice using a 1 mL syringe, 100 µL per mouse, with about 3.0×10⁶ cells for each mouse.

When the average tumor size reached approximately 89 mm³, the mice with moderate tumor sizes were selected. The animals were randomly allocated into test groups according to the tumor volumes, with 6 animals for each group, and the day of grouping was defined as D0, at which day the mice began to receive intraperitoneal injection (i.p.) of drugs, with the specific dosing regimen shown in Table 1.

**Table 1. Dosing regimen**

| Group | Drug | Dose (mg/kg) | Dosing regimen |
|---|---|---|---|
| 1 | PBS | - | D0, D3, D6, D12, D19, D26, totally 6 times |
| 2 | IMM47 | 2.0 (first 3 times) | D0, D3, D6, D12, D19, D26, totally 6 times |
| | | 1.0 (last 3 times) | |
| 3 | tislelizumab (innovator drug) | 2.0 | D0, D3, D6, D12, D19, D26, totally 6 times |
| 4 | IMM47+ tislelizumab | 2.0+2.0 (first 3 times) | D0, D3, D6, D12, D19, D26, totally 6 times |
| | | 1.0+2.0 (last 3 times) | |

The tumor sizes were measured twice a week, and the mice were observed till D33. The mice from the vehicle group were euthanized on D26 as the tumors exceeded 3000 mm³. The tumor volume (TV) was calculated with TV=1/2×a×b², where the "a" represented the long diameter of the tumor and "b" represented the short diameter of the tumor.

The relative tumor volume (RTV) was calculated with RTV = Vₜ/Vᵢₙᵢₜᵢₐₗ × 100%, where Vᵢₙᵢₜᵢₐₗ represented the tumor size measured at the time of grouping (i.e., D0) and Vt represented the tumor size at each measurement. The relative tumor proliferation rate T/C (%) was calculated as T/C (%) = (T_{RTV}/C_{RTV}) × 100%, where T_{RTV} represented the relative tumor volume in the treatment group and C_{RTV} represented the relative tumor volume in the vehicle group. The tumor volume inhibition rate (TGI) was calculated as TGI = [1-(TVₜ-TVᵢₙᵢₜᵢₐₗ)/(CVₜ-CVᵢₙᵢₜᵢₐₗ)] × 100%, where TVt represented the tumor volume of the treatment group at each measurement and TVᵢₙᵢₜᵢₐₗ represented the tumor volume of the treatment group at the time of grouping, and CVt represented the tumor volume of the control group at each measurement and CVᵢₙᵢₜᵢₐₗ represented the tumor volume of the control group at the time of grouping. The animal body weight change rate (BWC) was calculated as (BW_{final} - BWᵢₙᵢₜᵢₐₗ)BWᵢₙᵢₜᵢₐₗ × 100%, where BWᵢₙᵢₜᵢₐₗ represented the animal body weight at the time of grouping, and BW_{final} represented the animal body weight at each measurement. The tumor weight inhibition rate (IR) was calculated as (W_{C}-W_{T})/W_{C} × 100%, where W_{C} represented the tumor weight of the control group and W_{T} represented the tumor weight of the treatment group. The raw data were measured and recorded, and analysis was done based on the raw data. The analysis results were expressed as mean and standard error (Mean ± SEM), and the difference in tumor volume between the control group and the administration group was analyzed using T-test, with p < 0.05 indicating a statistical difference.

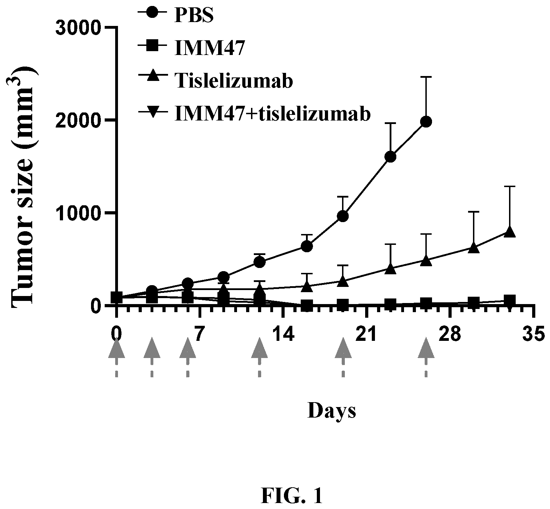

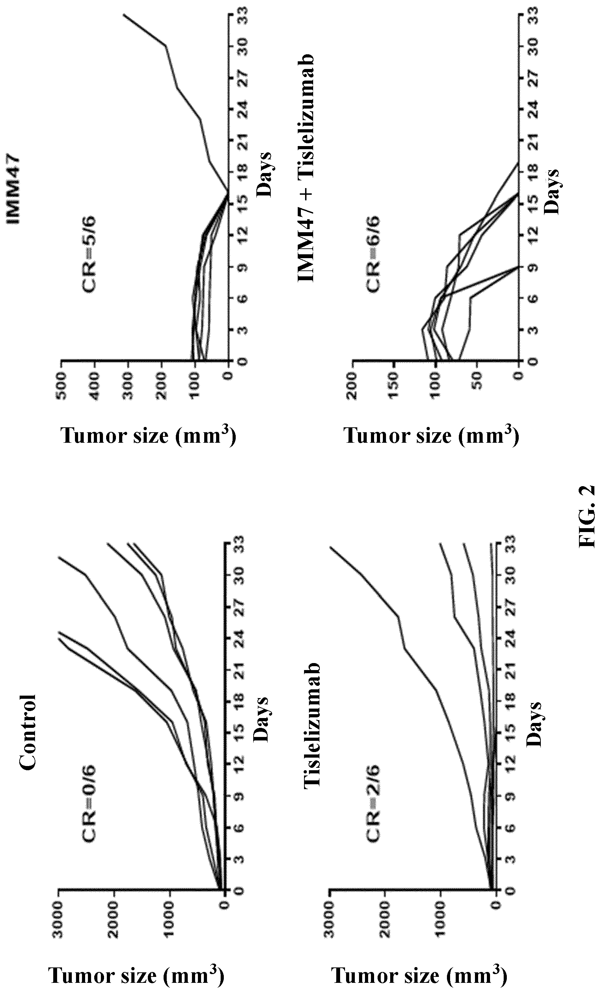

FIG. 1 showed that tislelizumab had some anti-tumor effects, and the IMM47 administration alone and IMM47 + tislelizumab combination use provided the best effects. From the tumor volume change of individual mice in FIG. 2, the anti-tumor effect of IMM47 + tislelizumab co-administration was even better than that of IMM47 administration alone. In particular, the complete remission (CR) reached 100% in the IMM47 + tislelizumab co-administration group, and the CR in the IMM47 alone group was slightly lower.

It can be seen that IMM47 and tislelizumab acted in a synergistic manner on tumors such as colon cancer, resulting in the complete elimination of tumor cells.

### Example 2. In vivo anti-tumor efficacy of IMM47 in combination with Opdivo^{®} or Keytruda^{®}

The MC38-hCD24/hPD-L1 cells were cultured in high-sugar DMEM medium containing 10% inactivated fetal bovine serum in an incubator at 37°C under 5% CO₂, and the cells were separated into flasks and passaged every 2 to 3 days when the confluency reached 80%.

The MC38-hCD24/hPD-L1 cells were collected at the log phase and resuspended in PBS for cell counting, and the cell density was adjusted to 3.0×10⁷/mL. The cell suspensions were inoculated subcutaneously into the right flanks of 6-8-week-old C57BL/6-hPD-1 mice using a 1 mL syringe, 100 µL per mouse, with about 3.0×10⁶ cells for each mouse.

When the average tumor size reached approximately 90 mm³, the mice with moderate tumor sizes were selected. The animals were randomly allocated into test groups according to the tumor volumes, with 6 animals for each group, and the day of grouping was defined as D0, at which day the mice began to receive intraperitoneal injection (i.p.) of drugs, with the specific dosing regimen shown in Table 2.

**Table 2. Dosing regimen**

| Group | Drug | Dose (mg/kg) | Dosing regimen |
|---|---|---|---|
| 1 | PBS | - | D0, D10, D20, totally 3 times |
| 2 | IMM47 | 0.5 | D0, D10, D20, totally 3 times |
| 3 | Opdivo^{®} (innovator drug) | 2.0 | D0, D10, D20, totally 3 times |
| 4 | Keytruda^{®} (innovator drug) | 2.0 | D0, D10, D20, totally 3 times |
| 5 | IMM47+Opdivo^{®} | 0.5+2.0 | D0, D10, D20, totally 3 times |
| 6 | IMM47+Keytruda^{®} | 0.5+2.0 | D0, D10, D20, totally 3 times |

Tumor sizes were measured twice a week until D24. Tumor volume (TV) was calculated as TV = 1/2 × a × b², where the "a" represented the long diameter of the tumor and "b" represented the short diameter of the tumor.

The relative tumor volume (RTV), relative tumor proliferation rate T/C (%), tumor volume inhibition rate (TGI), animal body weight change (BWC), and tumor weight inhibition rate (IR) were calculated as above. The raw data were measured and recorded, and analysis was done based on the raw data. The analysis results were expressed as mean and standard error (Mean ± SEM), and the difference in tumor volume between the control group and the administration group was analyzed using T-test, with p < 0.05 indicating a statistical difference.

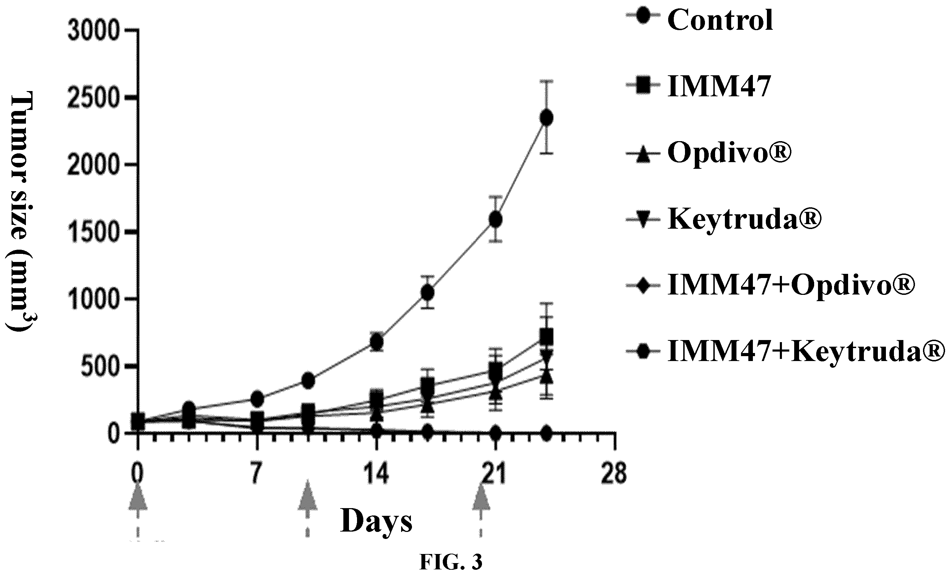

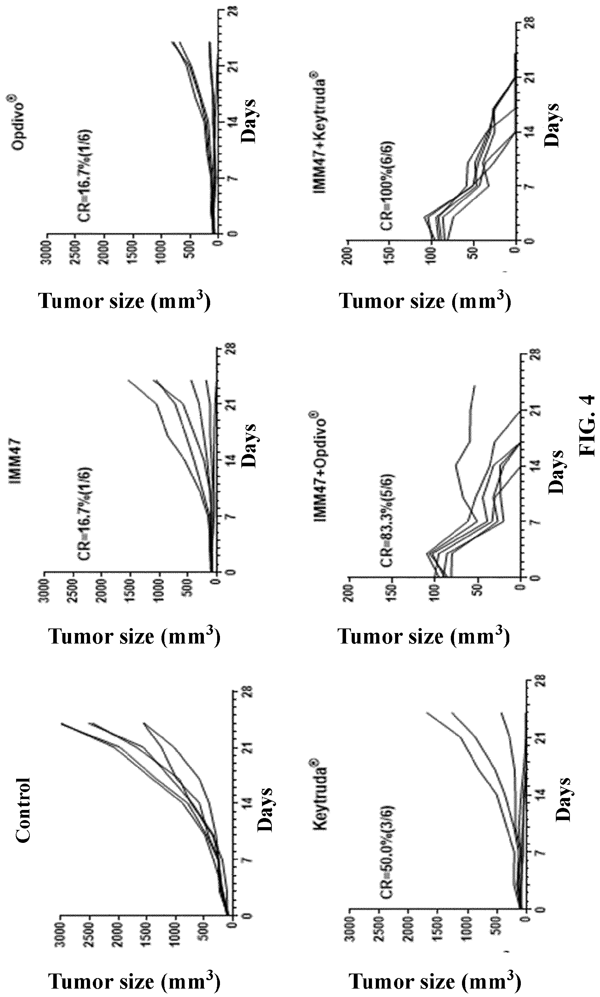

It can be seen from FIG. 3 that IMM47 administration alone, Opdivo^{®} administration alone, and Keytruda^{®} administration alone were effective in inhibiting tumor growth, whereas IMM47+ Opdivo^{®} co-administration, and IMM47+ Keytruda^{®} co-administration resulted in the complete elimination of tumor tissues. Further from the changes of individual tumors in FIG. 4, it can be seen that the complete remission rate (CR) was quite high in the IMM47-Opdivo^{®} or -Keytruda^{®} combination group, which was significantly higher than that of the other groups, indicating that the combination use inhibited tumor growth in a synergistic manner.

It can be seen from the results of Example 1 and Example 2 that, the IMM47 administration alone at the high dose showed high anti-tumor potency, which may have masked the synergistic effect to certain extent when IMM47 was used in combination with the anti-PD-1 antibody; and when the dose of IMM47 was reduced, it became obvious that IMM47 synergized with the anti-PD-1 antibody. Thus, the combination use of IMM47 with the anti-PD-1 antibody may achieve superior anti-tumor effect with even lower dose(s).

### Example 3. In vivo anti-tumor efficacy of IMM47 in combination with anti-PD-L1 antibody

The *in vivo* anti-tumor efficacy was further measured for IMM47 in combination with the PD-L1 antibodies, Tecentriq^{®} atezolizumab or IMM2515H.

The MC38-hCD24/hPD-L1 cells were cultured in high-sugar DMEM medium containing 10% inactivated fetal bovine serum in an incubator at 37°C under 5% CO₂, and the cells were separated into flasks and passaged every 2 to 3 days when the confluency reached 80%.

The MC38-hCD24/hPD-L1 cells were collected at the log phase and resuspended in PBS for cell counting, and the cell density was adjusted to 3.0×10⁷/mL. The cell suspensions were inoculated subcutaneously into the right flanks of 6-8-week-old C57BL/6-hPD-1 mice using a 1 mL syringe, 100 µL per mouse, with about 3.0×10⁶ cells for each mouse.

When the average tumor size reached approximately 100 mm³, the mice with moderate tumor sizes were selected. The animals were randomly allocated into test groups according to the tumor volumes, with 6 animals for each group, and the day of grouping was defined as D0, at which day the mice began to receive intraperitoneal injection (i.p.) of drugs, with the specific dosing regimen shown in Table 3.

**Table 3. Dosing regimen**

| Group | Drug | Dose (mg/kg) | Dosing regimen |
|---|---|---|---|
| 1 | PBS | - | QW×4, totally 4 times |
| 2 | IMM47 | 0.5 | QW×4, totally 4 times |
| 3 | Tecentriq^{®} (innovator drug) | 3.0 | QW×4, totally 4 times |
| 4 | IMM2515H | 3.0 | QW×4, totally 4 times |
| 5 | IMM47+Tecentriq^{®} | 0.5+3.0 | QW×4, totally 4 times |
| 6 | IMM47+IMM2515H | 0.5+3.0 | QW×4, totally 4 times |

Tumor sizes were measured twice a week until D24. Tumor volume (TV) was calculated as TV = 1/2 × a × b², where the "a" represented the long diameter of the tumor and "b" represented the short diameter of the tumor.

The relative tumor volume (RTV), relative tumor proliferation rate T/C (%), tumor volume inhibition rate (TGI), animal body weight change (BWC), and tumor weight inhibition rate (IR) were calculated as above. The raw data were measured and recorded, and analysis was done based on the raw data. The analysis results were expressed as mean and standard error (Mean ± SEM), and the difference in tumor volume between the control group and the administration group was analyzed using T-test, with p < 0.05 indicating a statistical difference.

The information of the sequences in present application was summarized as follows.

| Description/ |
|---|
| Sequence/SEQ ID NO. |
| HV-CDR1 of IMM47C, IMM47 and IMM47H |
| GYSITSGYS (SEQ ID NO: 1) |
| HV-CDR2 of IMM47C, IMM47 and IMM47H |
| IHYSGST (SEQ ID NO: 2) |
| HV-CDR3 of IMM47C, IMM47 and IMM47H |
| ARGADYALDY (SEQ ID NO: 3) |
| LV-CDR1 of IMM47C, IMM47 and IMM47H |
| QSLLYSSNQKNY (SEQ ID NO: 4) |
| LV-CDR2 of IMM47C, IMM47 and IMM47H |
| WAS |
| LV-CDR3 of IMM47C, IMM47 and IMM47H |
| QQNFIYPLT (SEQ ID NO: 5) |
| Heavy chain variable region of IMM47 and IMM47C |
| DVQLQESGPDLVKPSQSLSLTCTVTGYSITSGYSWHWIRQFPGNKLEWMGYIH YSGSTKYNPSLKSRISITRDTSKNQFFLQLNSVTTEDTATYFCARGADYALDYW GQRTSVTVSS (SEQ ID NO: 6) |
| Light chain variable region of IMM47 |
| DIQMTQSPSSLSASVGDRVTITCKSSQSLLYSSNQKNYLAWYQQKPGKAPKLLI YWASTRESGVPSRFSGSGSGTDFTLTISSLQPEDFATYYCQQNFIYPLTFGGGTK VELK (SEQ ID NO: 7) |
| Heavy chain constant region |
| ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHT CPPCPAPELLGGPSVFLFPPKPKDTLMISRTPEVTCVVVDVSHEDPEVKFNWYV DGVEVHNAKTKPREEQYNATYRVVSVLTVLHQDWLNGKEYKCKVSNKALPA PIAATISKAKGQPREPQVYTLPPSREEMTKNQVSLTCLVKGFYPSDIAVEWESN GQPENNYKTTPPVLDSDGSFFLYSKLTVDKSRWQQGNVFSCSVMHEALHNHY TQKSLSLSPGK (SEQ ID NO: 8) |
| Light chain constant region |
|  (SEQ ID NO: 9) |
| Light chain variable region of IMM47C |
| DIVMSQSPSSLAVSVGEKVTMSCKSSQSLLYSSNQKNYLAWYQQKPGHSPKLLI YWASTRESGVPDRFTGSGSGTDFTLTISSVKAEDLAVYYCQQNFIYPLTFGAGT KLELK (SEQ ID NO: 10) |
| Heavy chain variable region of IMM47H |
| DVQLQESGPGLVKPSETLSLTCTVSGYSITSGYSWHWIRQPPGKGLEWIGYIHYS GSTKYNPSLKSRVTISVDTSKNQFSLKLSSVTAADTAVYYCARGADYALDYWG QRTSVTVSS (SEQ ID NO: 11) |
| Light chain of IMM47H |
| DIVMTQSPDSLAVSLGERATINCKSSQSLLYSSNQKNYLAWYQQKPGQPPKLLI YWASTRESGVPDRFSGSGSGTDFTLTISSLQAEDVAVYYCQQNFIYPLTFGGGT KVELK (SEQ ID NO: 12) |
| HV-CDR1 of IMM2515H |
| GYTFTSNW (SEQ ID NO: 13) |
| HV-CDR2 of IMM2515H |
| IHPNSGSS (SEQ ID NO: 14) |
| HV-CDR3 of IMM2515H |
| ARSYYGSSPYYFDY (SEQ ID NO: 15) |
| LV-CDR1 of IMM2515H |
| QDIINY (SEQ ID NO: 16) |
| LV-CDR2 of IMM2515H |
| YTS |
| LV-CDR3 of IMM2515H |
| QQGDTLPWT (SEQ ID NO: 17) |
| Heavy chain variable region of IMM2515H |
| QVQLVQSGAEVKKPGASVKVSCKASGYTFTSNWMHWVRQAPGQGLEWMGM IHPNSGSSNYNEKFKSRVTMTRDTSTSTVYMELSSLRSEDTAVYYCARSYYGSS PYYFDYWGQGTLVTVSS (SEQ ID NO: 18) |
| Light chain variable region of IMM2515H |
| DIQMTQSPSSLSASVGDRVTITCRASQDIINYLNWYQQKPGKAPKLLIYYTSRLH SGVPSRFSGSGSGTDFTFTISSLQPEDIATYYCQQGDTLPWTFGQGTKVEIK (SEQ ID NO: 19) |

While the disclosure has been described above in connection with one or more embodiments, it should be understood that the disclosure is not limited to those embodiments. The description in the disclosure is intended to cover all modifications, and equivalents, as may be included within the spirit and scope of the appended claims. All references cited herein are incorporated by reference in their entirety.

## Claims

1. A composition, comprising:
i) an antibody or an antigen-binding portion thereof that binds specifically to CD24, and
ii) an antibody or an antigen-binding portion thereof capable of blocking PD-1-PD-L1 binding or PD-1-PD-L1 interaction,
wherein the antibody or the antigen-binding portion thereof that binds specifically to CD24 comprises a heavy chain variable region and a light chain variable region, wherein the heavy chain variable region comprises a HV-CDR1, a HV-CDR2, and a HV-CDR3, and the light chain variable region comprises a LV-CDR1, a LV-CDR2, and a LV-CDR3, wherein the HV-CDR1, the HV-CDR2, the HV-CDR3, the LV-CDR1, the LV-CDR2, and the LV-CDR3 comprise the amino acid sequences GYSITSGYS (SEQ ID NO: 1), IHYSGST (SEQ ID NO: 2), ARGADYALDY (SEQ ID NO: 3), QSLLYSSNQKNY (SEQ ID NO: 4), WAS, and QQNFIYPLT (SEQ ID NO: 5), respectively.

2. The composition of claim 1, wherein the heavy chain variable region and the light chain variable region of the antibody or the antigen-binding portion thereof that binds specifically to CD24 comprise the amino acid sequences of 1) SEQ ID NOs: 6 and 7, respectively; 2) SEQ ID NOs: 6 and 10, respectively; or 3) SEQ ID NOs: 11 and 12, respectively.

3. The composition of claim 1, wherein the antibody or the antigen-binding portion thereof that binds specifically to CD24 further comprises a heavy chain constant region with FcR binding ability.

4. The composition of claim 3, wherein the heavy chain constant region with FcR binding ability is human IgG1 heavy chain constant region.

5. The composition of claim 1, wherein the antibody or the antigen-binding portion thereof that binds specifically to CD24 comprises a heavy chain constant region and a light chain constant region, wherein the heavy chain constant region comprises the amino acid sequence of SEQ ID NO: 8 and the light chain constant region comprises the amino acid sequence of SEQ ID NO: 9.

6. The composition of claim 1, wherein the antibody or the antigen-binding portion thereof capable of blocking PD-1-PD-L1 binding or PD-1-PD-L1 interaction is
an antagonistic anti-PD-1 antibody or an antigen-binding portion thereof that binds specifically to PD-1, or
an antagonistic anti-PD-L1 antibody or an antigen-binding portion thereof that binds specifically to PD-L1.

7. The composition of claim 6, wherein the antagonistic anti-PD-1 antibody is selected from the group consisting of tislelizumab, nivolumab, and pembrolizumab.

8. The composition of claim 6, wherein the antagonistic anti-PD-L1 antibody is selected from the group consisting of atezolizumab and IMM2515H,
wherein IMM2515H comprises a heavy chain variable region and a light chain variable region, wherein the heavy chain variable region comprises a HV-CDR1, a HV-CDR2, and a HV-CDR3, and the light chain variable region comprises a LV-CDR1, a LV-CDR2, and a LV-CDR3, wherein the HV-CDR1, the HV-CDR2, the HV-CDR3, the LV-CDR1, the LV-CDR2 and the LV-CDR3 comprise amino acid sequences GYTFTSNW (SEQ ID NO: 13), IHPNSGSS (SEQ ID NO: 14), ARSYYGSSPYYFDY (SEQ ID NO: 15), QDIINY (SEQ ID NO: 16), YTS and QQGDTLPWT (SEQ ID NO: 17), respectively.

9. Use of an antibody or an antigen-binding portion thereof that binds specifically to CD24, and an antibody or an antigen-binding portion thereof capable of blocking PD-1-PD-L1 binding or PD-1-PD-L1 interaction, in preparation of a medicament for cancer treatment, wherein the antibody or the antigen-binding portion thereof that binds specifically to CD24 comprises a heavy chain variable region and a light chain variable region, wherein the heavy chain variable region comprises a HV-CDR1, a HV-CDR2, and a HV-CDR3, and the light chain variable region comprises a LV-CDR1, a LV-CDR2, and a LV-CDR3, wherein the HV-CDR1, the HV-CDR2, the HV-CDR3, the LV-CDR1, the LV-CDR2, and the LV-CDR3 contain the amino acid sequences GYSITSGYS (SEQ ID NO: 1), IHYSGST (SEQ ID NO: 2), ARGADYALDY (SEQ ID NO: 3), QSLLYSSNQKNY (SEQ ID NO: 4), WAS, and QQNFIYPLT (SEQ ID NO: 5), respectively.

10. The use of claim 9, wherein the heavy chain variable region and the light chain variable region of the antibody or the antigen-binding portion thereof that binds specifically to CD24 comprise the amino acid sequences of 1) SEQ ID NOs: 6 and 7, respectively; 2) SEQ ID NOs: 6 and 10, respectively; or 3) SEQ ID NOs: 11 and 12, respectively.

11. The use of claim 9, wherein the antibody or the antigen-binding portion thereof that binds specifically to CD24 further comprises a heavy chain constant region with FcR binding ability.

12. The use of claim 9, wherein the antibody or the antigen-binding portion thereof that binds specifically to CD24 comprises a heavy chain constant region and a light chain constant region, wherein the heavy chain constant region comprises the amino acid sequence of SEQ ID NO: 8 and the light chain constant region comprises the amino acid sequence of SEQ ID NO: 9.

13. The use of claim 9, wherein the antibody or the antigen-binding portion thereof capable of blocking PD-1-PD-L1 binding or PD-1-PD-L1 interaction is
an antagonistic anti-PD-1 antibody or an antigen-binding portion thereof that binds specifically to PD-1, or
an antagonistic anti-PD-L1 antibody or an antigen-binding portion thereof that binds specifically to PD-L1.

14. The use of claim 13, wherein the antagonistic anti-PD-1 antibody is selected from the group consisting of tislelizumab, nivolumab, and pembrolizumab.

15. The use of claim 13, wherein the antagonistic anti-PD-L1 antibody is selected from the group consisting of atezolizumab and IMM2515H,
wherein IMM2515H comprises a heavy chain variable region and a light chain variable region, wherein the heavy chain variable region comprises a HV-CDR1, a HV-CDR2, and a HV-CDR3, and the light chain variable region comprises a LV-CDR1, a LV-CDR2, and a LV-CDR3, wherein the HV-CDR1, the HV-CDR2, the HV-CDR3, the LV-CDR1, the LV-CDR2 and the LV-CDR3 comprise amino acid sequences GYTFTSNW (SEQ ID NO: 13), IHPNSGSS (SEQ ID NO: 14), ARSYYGSSPYYFDY (SEQ ID NO: 15), QDIINY (SEQ ID NO: 16), YTS and QQGDTLPWT (SEQ ID NO: 17), respectively.

16. The use of claim 9, wherein the cancer is selected from the group consisting of gastric cancer, liver cancer, colon cancer, non-small cell lung cancer, small cell lung cancer, ovarian cancer, pancreatic cancer, breast cancer, cervical cancer, endometrial cancer, head and neck tumor, uroepithelial carcinoma, cholangiocarcinoma, bladder cancer, glioblastoma, acute lymphocytic leukemia (ALL), acute myeloid leukemia (AML), multiple myeloma, Hodgkin's lymphoma (HL) and non-Hodgkin's lymphoma (NHL).
